# EUROPEAN PATENT APPLICATION

(11) **EP 3 011 896 A1**
(43) Date of publication of application: **27.04.2016**
(21) Application number: 15157849.9
(22) Date of filing: 05.03.2015
(51) Int. Cl.: A61B 5/00, A61B 5/103, A61B 5/11, A47C 31/00

(54) **System for monitoring sitting posture in real-time using pressure sensors**

(30) Priority: 24.10.2014 KR 20140145198; 05.02.2015 KR 20150017744
(71) Applicant: Soonchunhyang University Industry Academy Cooperation Foundation, Asan-si, Chungcheongnam-do 336-745 (KR)
(72) Inventor: Min, Se-Dong, Chungcheongnam-do (KR)
(74) Representative: Appleyard Lees IP LLP

(57) **Abstract**

A system for monitoring a sitting posture in real-time using pressure sensors includes: a measurement unit including a first pressure sensor for sensing a pressure applied to a chair by the left thigh, a second pressure sensor for sensing a pressure applied to the chair by the right thigh, a third pressure sensor for sensing a pressure applied to the chair by the left buttock, a fourth pressure sensor for sensing a pressure applied to the chair by the right buttock, a fifth pressure sensor for sensing a pressure applied to the chair by the left back, and a sixth pressure sensor for sensing a pressure applied to the chair by the right back; a control unit for converting pressure values sensed by the first pressure sensor, the second pressure sensor, the third pressure sensor, the fourth pressure sensor, the fifth pressure sensor and the sixth pressure sensor into digital data and transmitting the converted data; and a monitoring unit for storing and displaying the pressure digital data transmitted from the control unit by the measured date and time.

## Description

### BACKGROUND OF THE INVENTION

### Field of the Invention

The present invention relates to a system for monitoring a sitting posture in real-time using pressure sensors, and more specifically, to a system for monitoring a sitting posture in real-time using pressure sensors, which can recognize a form of a sitting posture in real-time by separately sensing pressures respectively applied to a chair by the left thigh, the right thigh, the left buttock, the right buttock, the left back and the right back.

### Background of the Related Art

As working and learning environments are changed owing to development of computer science and advancement in economy, times living in a chair, rather than doing dynamic physical activities, have been abruptly increased. If a person maintains an incorrect sitting posture for a long time in these environments, the person may easily feel fatigue and suffer from low back pain.

The low back pain is a typical disease related to the spine, which is experienced by eighty per cents of the total population due to various causes and results from a poor posture as well as spinal damage.

Since the low back pain can be sufficiently lessened and improved through a treatment merely correcting the posture and building a good habit, rather than a recovery through a direct treatment, before curvature of the spine is worsened, systems for correcting the sitting posture are actively developed recently.

However, a conventional system has a problem in that an algorithm for acquiring or processing measurement information for determining a sitting posture is complicated, and a type of an incorrect posture is difficult to be determined in real-time.

The background technique of the present invention has been disclosed in Korean Patent Publication No. 10-2014-0032082 filed on March 14, 2014

### SUMMARY OF THE INVENTION

Therefore, the present invention has been made in view of the above problems, and it is an object of the present invention to provide a system for monitoring a sitting posture in real-time using pressure sensors, which can recognize a form of a sitting posture in real-time by separately sensing pressures respectively applied to a chair by the left thigh, the right thigh, the left buttock, the right buttock, the left back and the right back.

Technical problems to be solved by the present invention are not limited to the technical problems described above, and unmentioned other technical problems may be clearly understood by those skilled in the art from the following descriptions.

To accomplish the above object, according to one aspect of the present invention, there is provided a system for monitoring a sitting posture in real-time using pressure sensors, the system including: a measurement unit including a first pressure sensor for sensing a pressure applied to a chair by the left thigh, a second pressure sensor for sensing a pressure applied to the chair by the right thigh, a third pressure sensor for sensing a pressure applied to the chair by the left buttock, a fourth pressure sensor for sensing a pressure applied to the chair by the right buttock, a fifth pressure sensor for sensing a pressure applied to the chair by the left back, and a sixth pressure sensor for sensing a pressure applied to the chair by the right back; a control unit for converting pressure values sensed by the first pressure sensor, the second pressure sensor, the third pressure sensor, the fourth pressure sensor, the fifth pressure sensor and the sixth pressure sensor into digital data and transmitting the converted data; and a monitoring unit for storing and displaying the pressure digital data transmitted from the control unit by the measured date and time.

In one exemplary embodiment, the respective pressure sensors are arranged on a chair at positions arranged to correspond with the left thigh, right thigh, left buttock, right buttock, left back and right back. Here, the chair comprises a chair seat and a chair back. The thigh and buttock sensors are arranged on the chair seat and the back sensors are arranged on the chair back.

In the system for monitoring a sitting posture in real-time using pressure sensors, the monitoring unit may display a case in which the pressure applied to the chair by the left thigh is zero, the pressure applied to the chair by the right thigh is not zero and the pressure applied to the chair by the left back is not zero, or the pressure applied to the chair by the left thigh is not zero, the pressure applied to the chair by the right thigh is zero and the pressure applied to the chair by the left back is not zero as a posture crossing the left leg.

In one exemplary embodiment, the respective sensors are arranged to detect a zero pressure and a not zero pressure when the pressure sensor senses a pressure below and above a threshold pressure respectively. That is, the sensors may be arranged to sense a zero pressure, when the sensor senses a minimal pressure, below the preset threshold pressure and may be arranged to sense a not zero pressure when the sensed pressure exceeds the preset threshold pressure. Here, when the preset threshold pressure is set at zero rather than a minimal pressure, the system is arranged to sense a pressure below the threshold pressure when the pressure sensed by the sensors is zero and to sense a pressure above the threshold pressure when the pressure sensed by the sensors is not zero.

In the system for monitoring a sitting posture in real-time using pressure sensors, the monitoring unit may display a case in which the pressure applied to the chair by the left thigh is zero, the pressure applied to the chair by the right thigh is not zero and the pressure applied to the chair by the left back is zero, or the pressure applied to the chair by the left thigh is not zero, the pressure applied to the chair by the right thigh is zero and the pressure applied to the chair by the left back is zero as a posture crossing the right leg.

In the system for monitoring a sitting posture in real-time using pressure sensors, the monitoring unit may display a case in which the pressure applied to the chair by the left thigh is not zero or the pressure applied to the chair by the right thigh is zero, and the pressure applied to the chair by the left thigh is zero or the pressure applied to the chair by the right thigh is not zero, and the pressure applied to the chair by the left buttock is zero and the pressure applied to the chair by the right buttock is zero as a posture pushing forward the buttocks.

In the system for monitoring a sitting posture in real-time using pressure sensors, the monitoring unit may display a case in which the pressure applied to the chair by the left thigh is not zero or the pressure applied to the chair by the right thigh is zero, and the pressure applied to the chair by the left thigh is zero or the pressure applied to the chair by the right thigh is not zero, and the pressure applied to the chair by the left buttock is not zero or the pressure applied to the chair by the right buttock (RH) is not zero, and the pressure applied to the chair by the left back is zero and the pressure applied to the chair by the right back is zero as a posture bending down the upper body.

In the system for monitoring a sitting posture in real-time using pressure sensors, the monitoring unit may display a case in which the pressure applied to the chair by the left thigh is not zero or the pressure applied to the chair by the right thigh is zero, and the pressure applied to the chair by the left thigh is zero or the pressure applied to the chair by the right thigh is not zero, and the pressure applied to the chair by the left buttock is not zero or the pressure applied to the chair by the right buttock (RH) is not zero, and the pressure applied to the chair by the left back is not zero and the pressure applied to the chair by the right back is not zero as a correct posture.

In exemplary embodiments, when pressure is applied to the chair, the respective sensors sense a pressure.

According to one exemplary embodiment the system is arranged to operate the method of the background technique as described in Korean Patent Publication No. 10-2014-0032082 filed on March 14, 2014, the features of the background technique being hereby incorporated by reference, and the features of which may therefore be combined with one or more of the exemplary embodiments.

### BRIEF DESCRIPTION OF THE DRAWINGS

FIG. 1 is a block diagram showing a system for monitoring a sitting posture in real-time using pressure sensors according to an embodiment of the present invention.
FIG. 2 is a mimic diagram showing a chair arranging a measurement unit of a system for monitoring a sitting posture in real-time using pressure sensors according to an embodiment of the present invention.
FIG. 3 is a view showing a posture classification tree applied to a system for monitoring a sitting posture in real-time using pressure sensors according to an embodiment of the present invention.

### DESCRIPTION OF SYMBOLS

### DETAILED DESCRIPTION OF THE PREFERRED EMBODIMENT

The preferred embodiments of the invention will be hereafter described in detail, with reference to the accompanying drawings.

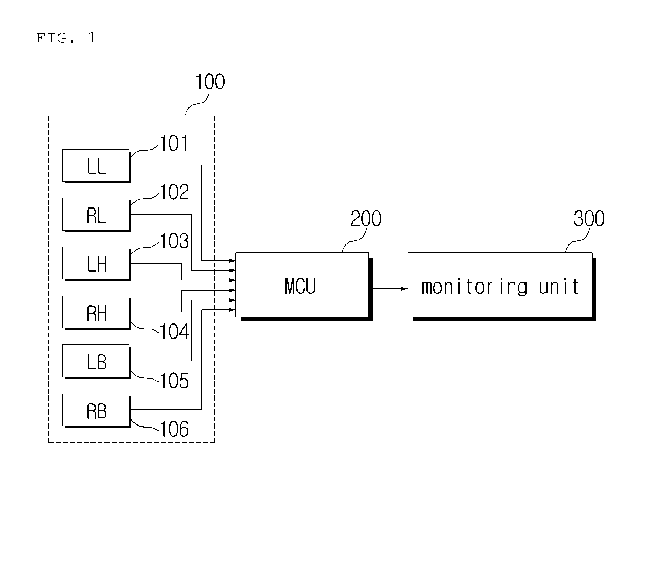

As shown in FIG. 1, a system for monitoring a sitting posture in real-time using pressure sensors according to an embodiment of the present invention may be configured to include a measurement unit 100, a control unit 200 and a monitoring unit 300.

Here, the measurement unit 100 includes a first pressure sensor 101 for sensing a pressure applied to a chair by the left thigh LL, a second pressure sensor 102 for sensing a pressure applied to the chair by the right thigh RL, a third pressure sensor 103 for sensing a pressure applied to the chair by the left buttock LH, a fourth pressure sensor 104 for sensing a pressure applied to the chair by the right buttock RH, a fifth pressure sensor 105 for sensing a pressure applied to the chair by the left back LB, and a sixth pressure sensor 106 for sensing a pressure applied to the chair by the right back RB.

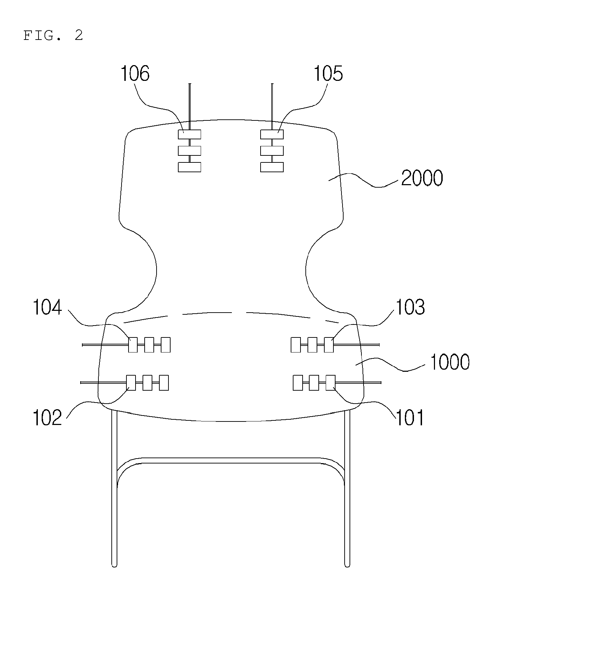

Specifically, as shown in FIG. 2, the first pressure sensor 101 is arranged at the left front of a chair seat 1000, the second pressure sensor 102 is arranged at the right front of the chair seat 1000, the third pressure sensor 103 is arranged at the left rear of the chair seat 1000, the fourth pressure sensor 104 is arranged at the right rear of the chair seat 1000, the fifth pressure sensor 105 is arranged at the upper left of a chair back 2000 connected to the chair seat 1000, and the sixth pressure sensor 106 is arranged at the upper right of the chair back 2000.

Meanwhile, the control unit 200 converts pressure values sensed by the first pressure sensor 101, the second pressure sensor 102, the third pressure sensor 103, the fourth pressure sensor 104, the fifth pressure sensor 105 and the sixth pressure sensor 106 into digital data and transmits the converted data to the monitoring unit 300 in a Bluetooth communication method.

In addition, the monitoring unit 300 receives the pressure digital data transmitted from the control unit 200 in the Bluetooth communication method and stores and displays the pressure digital data by the measured date and time. Here, the monitoring unit 300 may be configured of an Android-based application or an iPhone OS-based application.

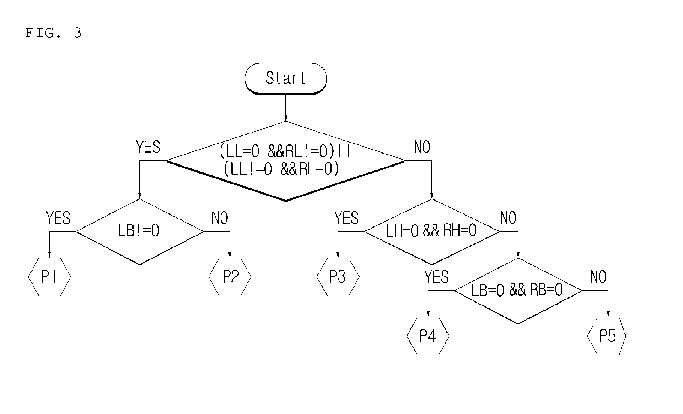

Hereinafter, a method of classifying a sitting form using the pressure digital data by the monitoring unit 300 is described with reference to FIG. 3.

The monitoring unit 300 displays a case in which the pressure applied to the chair by the left thigh (LL) is zero, the pressure applied to the chair by the right thigh (RL) is not zero and the pressure applied to the chair by the left back (LB) is not zero, or the pressure applied to the chair by the left thigh (LL) is not zero, the pressure applied to the chair by the right thigh (RL) is zero and the pressure applied to the chair by the left back (LB) is not zero as a posture crossing the left leg P1.

On the other hand, the monitoring unit 300 displays a case in which the pressure applied to the chair by the left thigh (LL) is zero, the pressure applied to the chair by the right thigh (RL) is not zero and the pressure applied to the chair by the left back (LB) is zero, or the pressure applied to the chair by the left thigh (LL) is not zero, the pressure applied to the chair by the right thigh (RL) is zero and the pressure applied to the chair by the left back (LB) is zero as a posture crossing the right leg P2.

In addition, the monitoring unit 300 displays a case in which the pressure applied to the chair by the left thigh (LL) is not zero or the pressure applied to the chair by the right thigh (RL) is zero, and the pressure applied to the chair by the left thigh (LL) is zero or the pressure applied to the chair by the right thigh (RL) is not zero, and the pressure applied to the chair by the left buttock (LH) is zero and the pressure applied to the chair by the right buttock (RH) is zero as a posture pushing forward the buttocks P3.

In addition, the monitoring unit 300 displays a case in which the pressure applied to the chair by the left thigh (LL) is not zero or the pressure applied to the chair by the right thigh (RL) is zero, and the pressure applied to the chair by the left thigh (LL) is zero or the pressure applied to the chair by the right thigh (RL) is not zero, and the pressure applied to the chair by the left buttock (LH) is not zero or the pressure applied to the chair by the right buttock (RH) is not zero, and the pressure applied to the chair by the left back (LB) is zero and the pressure applied to the chair by the right back (RB) is zero as a posture bending down the upper body P4.

In addition, the monitoring unit 300 displays a case in which the pressure applied to the chair by the left thigh (LL) is not zero or the pressure applied to the chair by the right thigh (RL) is zero, and the pressure applied to the chair by the left thigh (LL) is zero or the pressure applied to the chair by the right thigh (RL) is not zero, and the pressure applied to the chair by the left buttock (LH) is not zero or the pressure applied to the chair by the right buttock (RH) is not zero, and the pressure applied to the chair by the left back (LB) is not zero and the pressure applied to the chair by the right back (RB) is not zero as a correct posture P5.

Although six pressure sensors are used in the present invention, it is not limited thereto, but a sitting posture of a person can be classified and monitored as shown in the present invention by using a plurality of pressure sensors.

Since the system for monitoring a sitting posture in real-time using pressure sensors according to an embodiment of present invention may recognize a form of a sitting posture in real-time by separately sensing pressures respectively applied to a chair by the left thigh, the right thigh, the left buttock, the right buttock, the left back and the right back, whether or not the sitting posture is correct can be monitored in real-time.

While the present invention has been described in relation to preferred embodiments for illustrating the principle of the present invention, the present invention is not limited to the configurations and operations as is shown and described.

Rather, it is to be appreciated that those skilled in the art may make a plurality of changes or modifications of the present invention without departing from the scope and spirit of the attached claims.

Therefore, all the relevant modifications, changes and equivalents should be regarded within the scope of the present invention.

## Claims

1. A system for monitoring a sitting posture in real-time using pressure sensors, the system comprising:
a measurement unit including a first pressure sensor for sensing a pressure applied to a chair by a left thigh, a second pressure sensor for sensing a pressure applied to the chair by a right thigh, a third pressure sensor for sensing a pressure applied to the chair by a left buttock, a fourth pressure sensor for sensing a pressure applied to the chair by a right buttock, a fifth pressure sensor for sensing a pressure applied to the chair by a left back, and a sixth pressure sensor for sensing a pressure applied to the chair by a right back;
a control unit for converting pressure values sensed by the first pressure sensor, the second pressure sensor, the third pressure sensor, the fourth pressure sensor, the fifth pressure sensor and the sixth pressure sensor into digital data and transmitting the converted data; and
a monitoring unit for storing and displaying the pressure digital data transmitted from the control unit by measured date and time.

2. The system according to claim 1, wherein the monitoring unit displays a case in which the pressure applied to the chair by the left thigh is below a threshold pressure, the pressure applied to the chair by the right thigh is above a threshold pressure and the pressure applied to the chair by the left back is above a threshold pressure, or the pressure applied to the chair by the left thigh is above a threshold pressure, the pressure applied to the chair by the right thigh is below the threshold pressure and the pressure applied to the chair by the left back is above the threshold pressure as a posture crossing the left leg.

3. The system according to claim 1 or claim 2, wherein the monitoring unit displays a case in which the pressure applied to the chair by the left thigh is below a threshold pressure, the pressure applied to the chair by the right thigh is above a threshold pressure and the pressure applied to the chair by the left back is below a threshold pressure, or the pressure applied to the chair by the left thigh is above a threshold pressure, the pressure applied to the chair by the right thigh is below a threshold pressure and the pressure applied to the chair by the left back is below a threshold pressure as a posture crossing the right leg.

4. The system according to any of claims 1 to 3, wherein the monitoring unit displays a case in which the pressure applied to the chair by the left thigh is above a threshold pressure or the pressure applied to the chair by the right thigh is below a threshold pressure, and the pressure applied to the chair by the left thigh is below a threshold pressure or the pressure applied to the chair by the right thigh is above a threshold pressure, and the pressure applied to the chair by the left buttock is below a threshold pressure and the pressure applied to the chair by the right buttock is below a threshold pressure as a posture pushing forward the buttocks.

5. The system according to any of claims 1 to 4, wherein the monitoring unit displays a case in which the pressure applied to the chair by the left thigh is above a threshold pressure or the pressure applied to the chair by the right thigh is below a threshold pressure, and the pressure applied to the chair by the left thigh is below a threshold pressure or the pressure applied to the chair by the right thigh is above a threshold pressure, and the pressure applied to the chair by the left buttock is above a threshold pressure or the pressure applied to the chair by the right buttock (RH) is above a threshold pressure, and the pressure applied to the chair by the left back is below a threshold pressure and the pressure applied to the chair by the right back is below a threshold pressure as a posture bending down an upper body.

6. The system according to any of claims 1 to 5, wherein the monitoring unit displays a case in which the pressure applied to the chair by the left thigh is above a threshold pressure or the pressure applied to the chair by the right thigh is below a threshold pressure, and the pressure applied to the chair by the left thigh is below a threshold pressure or the pressure applied to the chair by the right thigh is above a threshold pressure, and the pressure applied to the chair by the left buttock is above a threshold pressure or the pressure applied to the chair by the right buttock (RH) is above a threshold pressure, and the pressure applied to the chair by the left back is above a threshold pressure and the pressure applied to the chair by the right back is above a threshold pressure as a correct posture.

7. The system according to any of claims 2 to 6 wherein the threshold pressure is zero.

8. The system according to any of claims 1 to 7, wherein the first pressure sensor is arranged at a left front of a chair seat, the second pressure sensor is arranged at a right front of the chair seat, the third pressure sensor is arranged at a left rear of the chair seat, the fourth pressure sensor is arranged at a right rear of the chair seat, the fifth pressure sensor is arranged at a upper left of a chair back connected to the chair seat, and the sixth pressure sensor is arranged at a upper right of the chair back.
